Europäisches Patentamt

European Patent Office

Office européen des brevets

(11) Numéro de publication: **0 125 940**
**B1**

(12) **FASCICULE DE BREVET EUROPEEN**

(45) Date de publication du fascicule du brevet: 07.10.87

(51) Int. Cl.⁴: **A 01 G 13/04, A 01 K 67/00**

(21) Numéro de dépôt: **84400547.0**

(22) Date de dépôt: **19.03.84**

(54) **Enceintes fermées employées comme serre, châssis de culture ou vivier et améliorant les conditions d'exploitation de ces structures.**

(30) Priorité: **22.03.83 FR 8304648**

(43) Date de publication de la demande:
**21.11.84 Bulletin 84/47**

(45) Mention de la délivrance du brevet:
**07.10.87 Bulletin 87/41**

(84) Etats contractants désignés:
**AT BE CH DE GB IT LI LU NL SE**

(56) Documents cités:
**FR-A-1 434 817**
**GB-A-1 580 934**
**GB-A-2 094 602**

(73) Titulaire: **Bourrié, André**
**19 avenue du Gal de Gaulle**
**F-78200 Mantes-la-Jolie (FR)**

(72) Inventeur: **Bourrié, André**
**19 avenue de Gal de Gaulle**
**F-78200 Mantes La Jolie (FR)**
Inventeur: **Maroselli, Jacques**
**18 allée André Maroselli**
**F-70300 Luxeuil Les Bains (FR)**

(74) Mandataire: **Madeuf, Claude Alexandre Jean et al**
**CABINET MADEUF 3, avenue Bugeaud**
**F-75116 Paris (FR)**

Courier Press, Leamington Spa, England.

EP 0 125 940 B1

## Description

On connaît déjà des enceintes destinées à la production intensive de plants en particulier à partir de graines, ces enceintes appelées châssis, petites serres, serres en verre ou en matière plastique transparente ou translucide sont largement utilisées pour les cultures de semis de plants, de fleurs ou analogues en assurant une protection de ces végétaux en cas de gel car ces enceintes permettent de maintenir une ambiance tiède et humide favorable à la germination, à la croissance ou à la culture végétale, voire même à l'élevage de petits animaux, mais ces enceintes, si elles ont donné d'assez bons résultats, nécessitent un travail long, délicat et devant être effectué juste en temps voulu. De plus, ces matériels résistent mal aux intempéries brutales telles que tempêtes, grosses chutes de neige, gelées très importantes. On est amené, en outre, par des moyens divers, compliqués et onéreux, d'une part, à assurer un arrosage périodique très surveillé en qualité et en quantité, et d'autre part, parfois à compléter, par un apport de chaleur, la température régnant à l'intérieur de ces enceintes.

Comme illustration de l'état de la technique, on peut indiquer que la demande de brevet GB—A—2 094 602 décrit une enceinte fermée employée comme serre formant un couvercle amovible présentant des rebords délimitant une partie centrale creuse percée d'au moins un trou pour l'arrosage du milieu de culture, cette enceinte étant réalisée en matière plastique convenable pour l'apport de chaleur.

On peut également prendre en considération le brevet FR—A—1 437 817 qui montre une enceinte fermée employée comme serre. Le bord du chapeau 16 et la paroi de l'enceinte 15 comportent tous deux une fente, respectivement 17 et 18. Ces centes peuvent se mettre en regard l'une et l'autre et, en faisant tourner le chapeau, il est possible de donner accès à plus ou moins d'air à l'intérieur de l'enceinte.

La présente invention apporte des perfectionnements importants à ces enceintes en permettant un réglage du fonctionnement très simple, ce qui diminue ainsi largement le travail et les frais d'entretien.

Conformément à l'invention, l'ensemble employé comme serre, châssis de culture ou vivier comportant au moins un couvercle et éventuellement une enceinte recouverte par ce couvercle, ladite enceinte formant bac ou ne comportant pas de fond, de manière à ce que l'ensemble recouvre totalement l'organisme vivant à protéger ou à élever soit directement dans le sol soit dans un milieu de culture placé dans le bac, cet ensemble constitué par au moins le couvercle amovible qui présente au moins une partie creuse centrale délimitée par des rebords et percée d'au moins un trou afin d'assurer un arrosage par un liquide contenu dans la partie en creux vers le milieu de culture est caractérisé en ce que les parois latérales extérieures du couvercle précité sont percées de trous qui sont normalement fermés par des bouchons de forme correspondante, bouchons présentant au moins une fente diamétrale permettant de régler la quantité d'air admise sous l'ensemble couvert et en ce qu'il est prévu, pour empêcher toute pénétration sous l'ensemble du liquide d'arrosage ou autre liquide, de placer dans la ou les parties en creux centrales des caches obturant les trous d'arrosage éventuellement en forme de fentes et en ce que les parois intérieures du couvercle de l'ensemble délimitant la partie en creux comportent une échelle graduée pour mesurer la quantité de liquide d'arrosage.

Suivant une autre caractéristique de l'invention, les bacs inférieurs sont bordés à leur partie supérieure de rebords percés de trous facilitant l'évacuation du liquide d'arrosage en surplus, ces rebords assurant la liaison entre le couvercle et le bac.

Diverses autres caractéristiques de l'invention ressortent d'ailleurs de la description détaillée qui suit.

Des formes de réalisation de l'objet de l'invention sont représentes, à titre d'exemples non limitatifs, aux dessins annexés.

La fig. 1 est une vue en perspective d'une première forme de réalisation de l'objet de l'invention.

La fig. 2 est une coupe transversale de l'enceinte de la fig. 1.

La fig. 3 est une coupe partielle, à grande échelle, montrant un détail de la fig. 1.

La fig 4 est une coupe suivant la ligne IV—IV de la fig. 1.

La fig. 5 montre en perspective une variante de réalisation de l'enceinte de la fig. 1.

La fig. 6 et 7 sont des vues en perspective des extrémités de l'enceinte de la fig. 5.

La fig. 8 montre en perspective une autre forme de réalisation de l'enceinte.

La fig. 9 est une vue en perspective d'une variante de la fig. 8.

La fig. 10 est en coupe une autre variante de la fig. 6.

La fig. 11 est en coupe une autre variante possible de la fig. 5.

La fig. 12 est une vue en perspective d'une variante de la fig. 10.

Les fig. 13, 14 et 15 montrent, en perspective et en coupe-élévation, de légères variantes ou des détails de réalisation employables sur les variantes des fig. 9, 10 et 11.

La fig. 15a est une vue en élévation de face d'un organe de maintien.

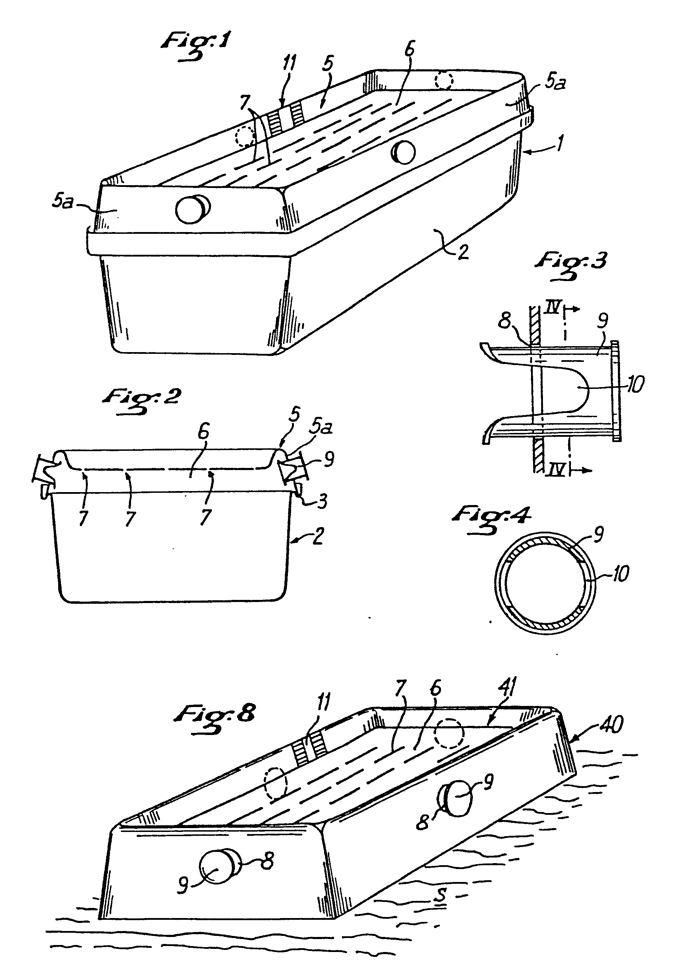

A la fig. 1, on a représenté une enceinte 1 constituée par un bac inférieur 2 de forme sensiblement parallélépipédique rectangle dont le bord supérieur 3 entourant le bac 2 sur ses quatre côtés a en section la forme d'un "S" horizontal (voir fig. 13). Des trous 4 sont en général percés dans le fond de l'"S" afin d'évacuer le surplus d'eau ou de liquide qui pourrait tomber des bords du couvercle 5 (voir fig. 2 et 3), couvercle qui présente en son centre une partie en creux 6 percée d'une multitude de trous ou fentes 7 permettant ainsi d'arroser d'eau ou d'eau de mélange d'engrais ou autre les plantes qui sont cultivées à l'intérieur du bac 2. Les ouvertures 7 ont des dimensions en rapport avec la surface à cultiver délimitée par les surfaces du brac 2.

De plus et comme cela est visible aux fig 1 et 2, le couvercle 5 est percé, sur ses parois latérales 5a, de trous 8 qui sont normalement fermés par des bouchons 9 de forme cylindrique mais qui comportent au moins une fente radiale 10 permettant de faire pénétrer plus ou moins d'air à l'intérieur de l'enceinte 1. On peut même en enfonçant complètement les bouchons 9 dans les trous 8 fermer ceux-ci entièrement en particulier pendant les époques où le gel peut abîmer les plants cultivés, à l'intérieur de l'enceinte 1. La partie en creux 6 du couvercle 5 présente, sur l'une de ses faces, une échelle graduée 11 permettant de mesurer la quantité de liquide placée dans le couvercle 5. Ainsi à l'intérieur de l'enceinte peut régner une température contrôlable à l'aide d'un instrument habituel mais également un arrosage suffisant pour les plantes qui s'y développent. Cet arrosage, du fait de la lenteur de la pénétration de l'eau dans l'enceinte 1 par les ouvertures 7, permet de différer ce travail qui peut dont être fait aisément et qui n'est plus une astreinte.

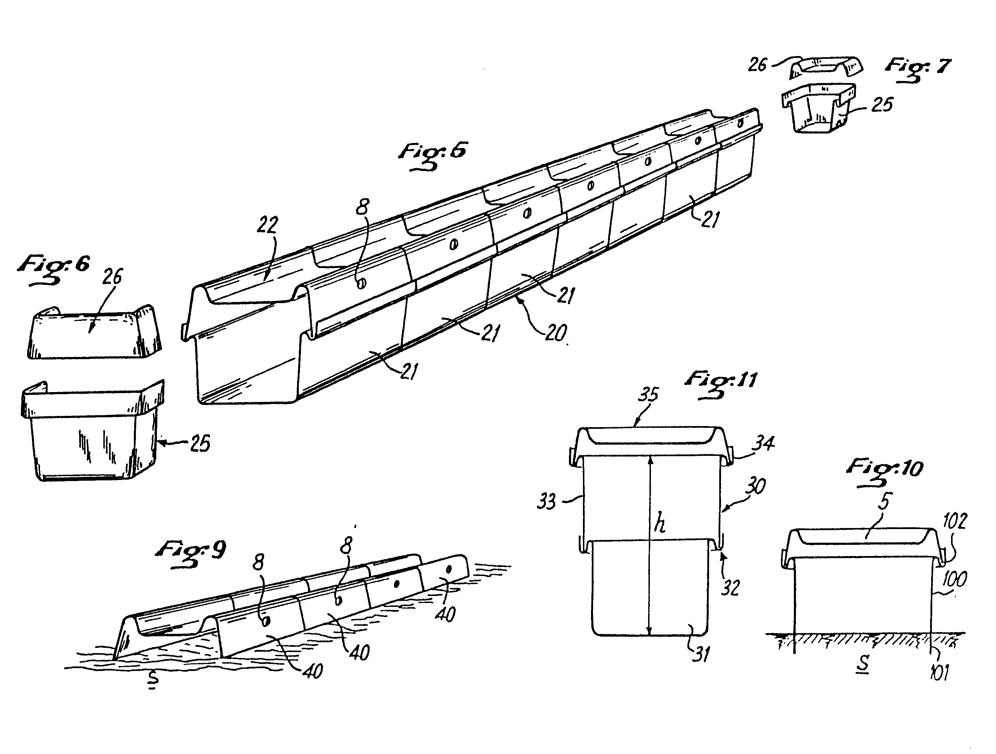

L'enceinte décrite ci-dessus est particulièrement destinée à des cultures en petites quantités par exemple des cultures personnelles de fleurs, mais lorsqu'on désire faire des cultures en plus grand nombre, on est amené à utiliser des enceintes beaucoup plus grandes du type de celles représentées à la fig. 5. Là, ces enceintes 20 comportent un bac inférieur 21 dont les extrémités sont libres et ouvertes afin de pouvoir accoler plusieurs bacs 21 les uns à côté des autres comme montré à la fig. 5. Les couvercles 22 sont aussi sans fermeture transversale et peuvent donc être placés les uns à côté des autres pour réaliser une longueur de bac désirée qui comporte tous les dispositifs décrits ci-dessus pour la fig. 1. Finalement, l'enceinte 20 peut être fermée à ses extrémités par des embouts inférieur 25 et supérieur 20 comme cela est représenté aux fig. 6 et 7, on peut donc avoir ainsi une enceinte d'un volume considérable permettant des cultures en grand nombre. Dans certains cas, lorsque les plants doivent avoir une hauteur importante, les enceintes 30 (voir fig. 11) sont constituées par un bac inférieur 31 à rebords 32 en "S" surmontés par une cage 33 à bords 34 en "S" recouverts d'un couvercle 35 du type sus-décrit. La hauteur h permet alors de cultiver des plants de grande hauteur.

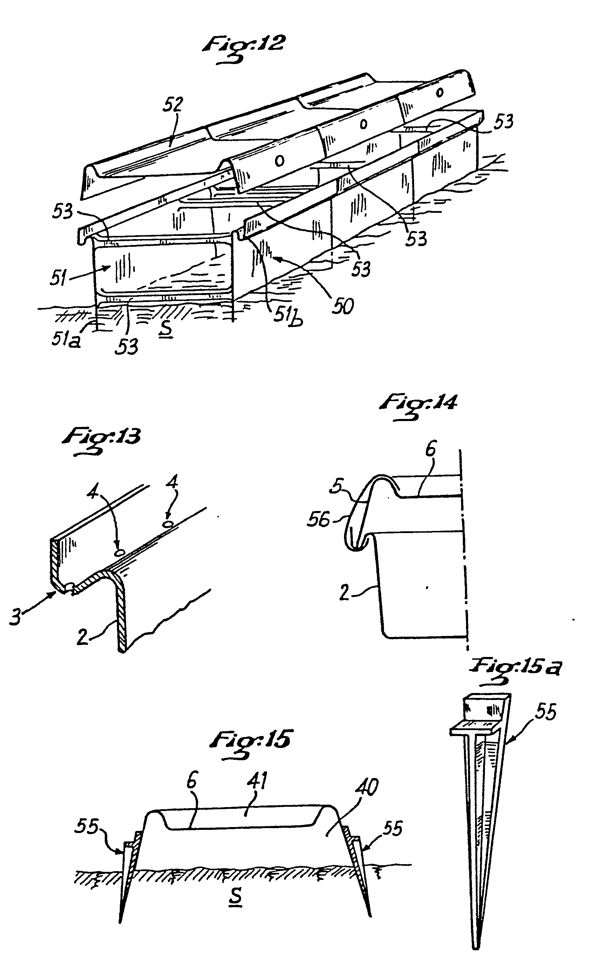

Par contre, lorsqu'on désire faire des cultures à même le sol "S", les bacs inférieurs ne sont pas nécessaires et on utilise alors des enceintes 40 (voir fig. 8, 9) qui sont composées exclusivement d'un couvercle 41 identique au couvercle décrit ci-dessus; ainsi on peut cultiver des plants directement dans le sol en les protégeant par un tunnel constitué par une ou plusieurs enceintes 40 sans avoir à assurer un travail long, pénible et contraignant.

A la fig. 9, on a représenté des couvercles 40 qui ne comportent pas d'éléments transversaux de façon à les accoler les uns aux autres, ces couvercles 40 pouvant être utilisés avec des embouts non représentés mais du genre de ceux décrits comme les embouts supérieurs 26 (voir fig. 6 et 7).

La fig. 10 montre une variante de la fig. 9 dans laquelle une enceinte sans fond 100 est placée directement sur le sol S et dont les bords inférieures 101 pénètrent dans le sol. Les bords supérieurs 102 sont conformes, comme décrit ci-dessus (voir fig. 2) et supportent un couvercle 5 identique à ceux décrits précédemment. On peut ainsi cultiver des plantes d'une certaine hauteur dans l'enceinte ainsi constituée.

A la fig. 12, l'enceinte 50 est destinée à permettre la culture sur le sol de plants qui sont protégés par des caissons 51 sensiblement parallélépipédiques rectangles dont le bord inférieur 51a est enfoncé dans le sol S et dont le bord supérieur 51b est conformé en S comme décrit ci-dessus avec possibilité d'avoir des perforations comme les trous 4 (voir fig. 13). Les couvercles 52 sont identiques à ceux décrits ci-dessus et, en particulier, à ceux décrits pour la fig. 9. Afin de raidir transversalement les caissons 51 ceux-ci comportent de distance en distance des traverses 53. Bien entendu, l'enceinte 50 formant tunnel peut également comporter à ses extrémités des embouts comme ceux décrits à la fig. 6, c'est-à-dire des embouts inférieurs mais sans fond et des embouts supérieurs identiques à ceux décrits sous la référence 26.

Dans le cas où l'on utilise les enceintes décrites aux fig. 8 et 9, on peut être amené à maintenir celles-ci dans le sol S (voir fig. 15) à l'aide de fiches 55 (fig. 15a) qui en pénétrant dans le sol S enserrent d'une manière sûre et certaine les parois latérales des enceintes 40 pour éviter ainsi tout déplacement de ces enceintes sous l'action du vent. Ces fiches 55 servant d'appui spécifique en position ouverte des enceintes qui ont l'avantage d'être ouvrantes sur chaque côté.

Finalement, et comme représenté à la fig 14, dans le cas des enceintes 1, 20, 30, 100, il est possible de verrouiller le couvercle 5 sur les bacs 2 par des griffes élastiques 56 maintenant ainsi fermés les couvercles 5 sur les bacs 2. On assure dont une sécurité vis-à-vis du vent.

Les enceintes sus-décrites peuvent être réalisées en toute matière mais elles le sont en général en matière plastique rigide, transparente ou translucidé.

La description ci-dessus permet d'indiquer que l'on peut réaliser une économie importante de main-d'oeuvre car l'arrosage se fait sans ouvrir les enceintes puisque les couvercles 5 permettant une alimentation en eau ou une répartition correcte. De plus, l'arrosage peut être fait à température déterminée et lorsque les cultures ont lieu à l'air libre, c'est-à-dire lorsque les enceintes sont directement posées sur le sol, l'eau de pluie est recueillie complètement, sans perte. La protection est, de plus, assurée contre le grêle, puisque ces enceintes résistent parfaitement à des chutes importantes de grêle.

Ces enceintes sont réalisées en matériaux légers et permettent cependant le transport rapide et facile lorsqu'il est nécessaire de les changer de place.

Dans certains cas, les enceintes décrites aux fig. 1, 2 et 5 peuvent être réalisées de manière à être parfaitement étanches et dont pouvoir contenir de l'eau pour le production d'animaux tels que les poissons (pisciculture), poissons décoratifs ou des poissons destinés à repeupler les rivières. Les couvercles formant cuvette permettent ainsi de distribuer la nourriture en quantité suffisante et pendant un certain laps de temps en réglant correctement la grosseur ou la largeur des trous ou fentes 7.

Comme il est décrit ci-dessus, les fentes ou trous 7 percés dans la surface en creux 6 des couvercles 5 ayant en général la forme d'incision longitudinale discontinue sont généralement identiques en longueur et alignés afin de conserver au convercle 5 le maximum d'effet de plaque nécessaire à la grande robustesse de la structure. De plus, dans le cas où l'eau de pluie est prohibée pendant une certaine période ou pour des espèces particulières, il est possible de prévoir des caches en forme de feuilles plastiques à bords légèrement rabattus qui viennent s'ajuster dans les couvercles 5 pour former parapluie.

Les enceintes sus-décrites permettent de capter l'intégralité de l'ensoleillement et de ce fait elles peuvent aussi souvent se substituer aux grandes serres classiques à ambiance.

On obtient ainsi, d'une part, un abaissement de l'investissement et, d'autre part, une éconimie d'énergie. Finalement, le taux d'intervention pour les cultures ainsi faites est extrêmement limité et le nombre du personnel bien moindre.

**Revendications**

1. Ensemble employé comme serre, châssis de culture ou vivier comportant au moins un couvercle (5, 22, 35, 41, 52) et éventuellement une enceinte (1, 20, 30, 40, 50, 100) recouverte par ce couvercle, ladite enceinte formant bac (2, 21, 31) ou ne comportant pas du fond, de manière à ce que l'ensemble recouvre totalement l'organisme vivant à protéger ou à élever soit directement dans le sol soit dans un milieu de culture placé dans le bac, cet ensemble constitué par au moins le couvercle (5, 22, 35, 41, 52) amovible qui présente au moins une partie creuse centrale (6) délimitée par des rebords (5a) et percée d'au moins un trou (7) afin d'assurer un arrosage par un liquide contenu dans la partie en creux (6) vers le milieu de culture, caractérisé en ce que les parois latérales extérieures du couvercle (5, 22, 35, 41, 52) précité sont percées de trous (8) qui sont normalement fermés par des bouchons (9) de forme correspondante, bouchons présentant au moins une fente diamétrale (10) permettant de régler la quantité d'air admise sous l'ensemble couvert, et en ce qu'il est prévu, pour empêcher toute pénétration sous l'ensemble du liquide d'arrosage ou autre liquide, de placer dans la ou les parties en creux centrales (6) des caches obturant les trous d'arrosage (7) éventuellement en forme de fentes, et en ce que les parois intérieures du couvercle (5, 22, 35, 41, 52) de l'ensemble délimitant la partie en creux (6) comportent une échelle graduée (11) pour mesurer la quantité de liquide d'arrosage.

2. Ensemble suivant la revendication 1, caractérisé en ce que les bacs (2) sont bordés à leur partie supérieure de rebords (3) percés de trous (4) facilitant l'évacuation du liquide d'arrosage en surplus, ces rebords (3) assurant la liaison entre le couvercle (5) et le bac (2).

3. Ensemble suivant la revendication 2, caractérisé en ce qu'il est prévu des griffes élastiques (56) maintenant les couvercles (5) sur les bacs (2).

4. Ensemble suivant la revendication 1, caractérisé en ce que lorsque les couvercles (5, 22, 35, 41, 52) sont placés directement sur le sol "S", leur bord périphérique est enfoncé sur le sol "S" et maintenu pour la résistance au vent par des fiches spécifiques (55) enserrant ces couvercles (5, 22, 25, 41, 52).

5. Ensemble suivant l'une des revendications 1 à 4, caractérisé en ce qu'elle est réalisée en matière imputrescible, légère, transparente ou translucide pour permettre de recueillir un ensoleillement maximum.

6. Ensemble suivant la revendication 1, caractérisé en ce que soit les enceintes (40, 50) sans fond, soit les bacs (2, 21, 31) sont ouverts à leurs deux extrémités transversales pour permettre de les placer dans le prolongement l'une de l'autre pour obtenir finalement des volumes plus ou moins grands de protection, les enceintes ou les bacs pouvant être rigidifiés transversalement par des traverses (53).

7. Ensemble suivant l'une des revendications 1 à 6, caractérisé en ce que les bacs (31) supportent par leurs bords périphériques (32) des cages sans fond (30) recouvertes par les couvercles (35) afind de pouvoir cultiver des plants de grande hauteur.

8. Ensemble suivant l'une des revendications 1 à 7, caractérisé en ce que les bacs (100) sont sans fond et peuvent donc être engagés par leur périphérie directement dans le sol "S" puis converts par des enceintes type couvercles (5, 35).

9. Ensemble suivant l'une des revendications 1 à 8, caractérisé en ce qu'elle comporte des embouts transversaux (25, 26) permettant de fermer une longueur indéterminée de cette enceinte.

**Patentansprüche**

1. Geschlossene Räume verwendbar als Gewächshaus, Treibfenster oder Vivarium, die aus mindestens einem Deckel (5, 22, 35, 41, 52), und ggf. einem mit diesen Deckel abgedeckten Gehäuse (1, 20, 30, 40, 50, 100) bestehen, wobei dieses Gehäuse als Trog (2, 21, 31) ausgebildet ist bzw. keinen Grund aufweist, so dass die gesamte Vorrichtung den lebendigen, zu schützenden bzw. entweder unmittelbar im Erdboden oder in einem im Trog enthaltenen Nährboden zu züchtenden Organismus gänzlich abgedeckt ist, und diese Vorrichtung mindestens aus dem abnehmbaren Deckel (5, 22, 35, 41, 52) besteht, der mindestens einen mittleren hohlen Teil (6) aufweist, der durch erhöhte Ränder (5a) begrenzt ist und mindestens ein Loch (7) aufweist, um eine Berieselung der gezüchteten Organismen durch eine im hohlen Teil (6) enthaltene Flüssigkeit sicherzustellen, dadurch gekennzeichnet, dass die seitlichen Aussenwände des genannten Deckels (5, 22, 35, 41, 52) von Löchern (8) durchbohrt sind, die normalerweise mittels eine entsprechende Gestalt aufweisender Stöpsel (9) verschlossen werden, die mindestens einen diametralen Spalt (10) aufweisen, der es ermöglicht, die in die abgedeckte Vorrichtung eingeleitete Luftmenge einzustellen, dass es vorgesehen ist, um jedes Eindrigen der Berieselungsflüssigkeit bzw. einer anderen Flüssigkeit unter die Vorrichtung zu vermeiden, in den (die) mittleren hohlen Teil (c) (6) Blendmasken einzusetzen, welche die ggf. als Spalte ausgebildeten Berieselungslöcher (7) verschliessen, und dass die Innenwände des den hohlen Teil (6) begrenzenden Deckels (5, 22, 35, 41, 52) der Vorrichtung einen Massstab (11) aufweisen, der die Messung der zur Berieselung bestimmten Flüssigkeit ermöglicht.

2. Geschlossene Räume nach Anspruch 1, dadurch gekennzeichnet, dass die Tröge (2) in ihrem oberen Teil mit Rändern (3) umgeben sind, die mit Löchern (4) versehen sind, die die Abführung der überschüssigen Berieselungsflüssigkeit erleichtern, wobei diese Ränder (3) die Verbindung zwischen dem Deckel (5) und dem Trog (2) sicherstellen.

3. Geschlossene Räume nach Anspruch 2, dadurch gekennzeichnet, dass elastische Klauen (56) vorgesehen sind, die die Deckel (5) auf den Trögen (2) festhalten.

4. Geschlossene Räume nach Anspruch 1, dadurch gekennzeichnet, dass der Umfangsrad der unmittelbar auf dem Boden "S" gelegenen Deckel (5, 22, 35, 41, 52) in den Boden "S" eingeschlagen und zum Zweck des Widerstands gegen den Wind durch spezifische Belestigungsmittel (55) festgehalten wird, die diese Deckel (5, 22, 25, 41, 52) umschliessen.

5. Geschlossene Räume nach einem der Ansprüche 1 bis 4, dadurch gekennzeichnet, dass die Vorrichtung aus einem unverweslichen, leichten, durchsichtigen oder durchscheinenden Material hergestellt wird, das es ermöglicht, eine maximale Sonnenstrahlung aufzunehmen.

6. Geschlossene Räume nach Anspruch 1, dadurch gekennzeichnet, dass entweder die grundlosen Gehäuse (40, 50) oder die Tröge (2, 21, 31) an ihren beiden Enden offen sind, was die Möglichkeit bietet, sie nacheinander in einer Fluchtlinie zu setzen, um schliesslich mehr oder weniger grosse geschlossene Schutzräume zu bewerkstelligen, wobei die Gehäuse und die Tröge in der Querrichtung durch Querstege (53) versteift werden können.

7. Geschlossene Räume nach einem der Ansprüche 1 bis 6, dadurch gekennzeichnet, dass die Tröge (31) über ihren Umfangsräumer (32) grundlose Käfige (30) tragen, die mit den Deckeln (35) abgedeckt werden, damit Pflanzen grosser Höhe gezüchtet werden können.

8. Geschlossene Räume nach einem der Ansprüche 1 bis 7, dadurch gekennzeichnet, dass die Tröge (100) keinen Grund aufweisen, so dass sie an ihrem Umfang unmittelbar in den Erdboden "S" eingeschlagen und dann mit Gehäusen in der Gestalt von Deckeln (5, 35) gedeckt werden können.

9. Geschlossene Räume nach einem der Anschlüsse 1 bis 8, dadurch gekennzeichnet, dass die Vorrichtung Querteile (25, 26) aufweist, die es ermöglichen, eine beliebige Länge des Gehäuses zu schliessen.

**Claims**

1. Assembly used as greenhouse, cultivation frame or fish pond comprising at least one cover (5, 22, 35, 41, 52) and possibly an enclosure (1, 20, 30, 40, 50, 100) covered by this cover, said enclosure forming a tank (2, 21, 31) or comprising no bottom, so that the assembly covers totally the living organism to be protected or breeded either directly in the ground or in a culture medium placed in the tank, this assembly formed by at least the removable cover (5, 22, 35, 41, 52) which has at least one central recessed portion (6) formed by edges (5a) and pierced with at least one hole (7) so as to provide a watering by a liquid contained in the recessed portion (6) towards the culture medium, characterized in that the outer side walls of the above mentioned cover (5, 22, 35, 41, 52) are pierced with holes (8) which are normally closed by plugs (9) of a corresponding shape, which plugs have at least one diametral slot (10) enabling to regulate the quantity of air admitted under the covered assembly, and in that there is provided, for preventing any penetration under the assembly of the watering liquid or other liquid, to place, in the central recessed portion(s) (6), masks obturating the possibly slot-shaped watering holes, and in that the inner walls of the cover (5, 22, 35, 41, 52) of the assembly defining the recessed portion (6) comprise a graduated scale (11) for measuring the quantity of watering liquid.

2. Assembly according to claim 1, characterized in that the tanks (2) are bordered at their upper parts with edges (3) pierced with holes (4) facilitating the removal of the watering liquid in excess, these edges (3) ensuring the connection between the cover (5) and the tank (2).

3. Assembly according to claim 2, characterized in that there is provided resilient claws (56) maintaining the covers (5) onto the tanks (2).

4. Assembly according to claim 1, characterized in that, when the covers (5, 22, 35, 41, 52) are placed directly on the ground "S", their peripheral edge is entered onto the ground "S" and maintained for wind resisting by specific pegs (55) encompassing these covers (5, 22, 35, 41, 52).

5. Assembly according to one of claims 1 to 4, characterized in that it is made of a transparent or translucent, light, non putrescible material for enabling to collect a maximum amount of sunshine.

6. Assembly according to claim 1, characterized in that either the bottomless enclosures (40, 50), or the tanks (2, 21, 31) are opened at their two side ends for enabling to place them in line one with the other for finally obtaining more or less large protecting volumes, the enclosures or the tanks being transversely stiffenable by crossbeams (53).

7. Assembly according to one of claims 1 to 6, characterized in that the tanks (31) support by their peripheral edges (32) bottomless cages (30) covered by the covers (35) so as to be able to cultivate plants of a great height.

8. Assembly according to one of claims 1 to 7, characterized in that the tanks (100) are bottomless and can therefore be engaged by their periphery directly into the ground "S", then covered by cover type enclosures (5, 35).

9. Assembly according to one of claims 1 to 8, characterized in that it comprises tranverse endpieces (25, 26) enabling to close an undetermined length of this enclosure.

0 125 940

Fig:1

Fig:2

Fig:3

Fig:4

Fig:8

*Fig. 7*

26

25

*Fig. 6*

22
8
21
21
21
20
21
21

*Fig. 6*

26

25

2

0 125 940

*Fig. 11*

35
34
33
30
h
32
31

*Fig. 10*

5
102
100
S
101

*Fig. 9*

8
8
8
40
40
40
40
S

*Fig.12*

*Fig.13*

*Fig.14*

*Fig.15*

*Fig.15a*